# EUROPEAN PATENT APPLICATION

(11) **EP 2 594 257 A1**
(43) Date of publication of application: **22.05.2013**
(21) Application number: 11189610.6
(22) Date of filing: 17.11.2011
(51) Int. Cl.: A61K 9/00, A61K 9/70

(54) **Orodispersible films for the manufacturing of individualised medicine or for large scale production**

(71) Applicant: Labtec GmbH, 40764 Langenfeld (DE)
(72) Inventor: Breitenbach, Armin, 51371 Leverkusen (DE); Braun. Sebastian, 42929 Wermelskirchen (DE)
(74) Representative: von Renesse, Dorothea

(57) **Abstract**

The present invention pertains to oral applicable therapeutic dosage forms, in particular to orodispersible films. The present invention especially is directed to orodispersible films comprising a base layer substantially free of therapeutically active agents and a top layer comprising the desired therapeutically active agents. The present invention also concerns suitable base layers for such orodispersible films as well as therapeutical uses thereof and methods for manufacturing them.

## Description

### FIELD OF THE INVENTION

The present invention pertains to oral applicable therapeutic dosage forms, in particular to orodispersible films. The present invention also concerns suitable base layers for such orodispersible films as well as therapeutic uses thereof and methods for manufacturing them.

### TECHNICAL BACKGROUND

Orodispersible films (ODFs) disintegrate within seconds when placed on the tongue. Swallowing is not necessary. Therefore, they are an ideal oral dosage form i.e. for paediatrics and geriatrics. For the usual way of manufacturing the active pharmaceutical ingredient (API) is added to a polymer solution, which is casted, dried, cut to final film size and packaged.

The object of the present invention is to provide an ODF which is simple and economically to be manufactured.

This objective is achieved with an ODF which comprises a base layer which while casted is substantially free of any active pharmaceutical ingredient (API, drug substance) and a top layer with the API which is applied to the base layer after casting the base layer and prior to packaging the individualized films. This is advantageous in case of heat sensitive APIs, because the API is added after the main drying step. Furthermore API waste can be avoided, because the API is added after casting and, in a preferred embodiment, even after cutting. Using highly potent low-dose drugs providing content uniformity is easier. For these low dose high potent drugs the required safety areas for production can be smaller compared to standard manufacturing, which also refers to cleaning of only smaller manufacturing areas, since the API is no longer present in the large coating and converting equipment, but limited to the "printer".

In a preferred embodiment of the invention the top layer is printed onto the base layer. Printing on the base layer is highly flexible, because only a few types of base layer formulations are needed. Development of a new formulation for every API is not necessary.

Due to the high flexibility and the possibilities of small and large scale production, printing on base layers enables the manufacturing of individualized medicine as well as industrial mass production. Furthermore, this type of manufacturing enables the highest flexibility in production by means of batch size and dosing, since both will be determined by the printing step, while the essentially API free base layer can be produced and stored in large quantities upfront.

The challenge of applying a top layer printing on base layers is to provide a stable adhesion of the API containing top layer on the base layer. This requires the avoidance of early film disintegration during the application of the top layer e.g. by printing. However, the fast dissolving properties, which regularly characterize ODF shall be maintained.

### SUMMARY OF THE INVENTION

The present invention relates to orodispersible films which comprise applying (e.g. printing) a top layer containing an active pharmaceutical ingredient (API) onto a base layer which is manufactured without an API. In particular, the formulations of the base layer and the top layer are chosen so that a suitable adhesion of the top layer to the base layer is achieved and at the same time the base layer does not disintegrate during application of the top layer. This is especially challenging since on the one hand, the top layer is applied as solution or dispersion containing a solvent, and on the other hand, the base layer has to maintain its capability to quickly dissolve in the oral cavity when administered.

Thus, in a first aspect, the present invention provides an orodispersible film comprising a base layer and a top layer attached onto the base layer, wherein the base layer comprises at least one first film-forming substance, and wherein the top layer comprises at least one second film-forming substance and at least one oral applicable, therapeutically active agent.

In a second aspect, the present invention provides a base layer for use in an orodispersible film according to the first aspect of the invention, comprising at least one first film-forming substance.

In a third aspect, the present invention provides the use of a base layer according to the second aspect of the invention for the preparation of an orodispersible film, wherein a top formulation comprising at least one second film-forming substance and at least one oral applicable, therapeutically active agent is applied to the base layer.

In a fourth aspect, the present invention provides a method for preparing an orodispersible film, comprising the step of applying a top formulation comprising at least one second film-forming substance and at least one oral applicable, therapeutically active agent to the base layer according to the second aspect of the invention.

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the spirit and scope of the disclosed invention will become readily apparent to those skilled in the art from reading the following.

### DETAILED DESCRIPTION OF THE INVENTION

As used herein, the expressions defined herein are generally intended to preferably have the meanings as set forth below, except to the extent that the context in which they are used indicates otherwise.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

According to the invention the term "orodispersible film" refers to films which can be orally administered to a patient and which disintegrates or dissolves in the oral cavity of the patient. Disintegration or dissolution of the film preferably results from contact with salvia and in particular is achieved in a short period of time such as below 10 minutes, preferably below 5 minutes, 3 minutes, 2 minutes 90 seconds or 60 seconds. Preferably, the orodispersible film is a pharmaceutical dosage form and comprises at least one active pharmaceutical ingredient which in particular is suitable for oral administration.

The terms "active pharmaceutical ingredient", "pharmaceutically active agent" or "drug substance" are used interchangeably herein.

A "film-forming substance" according to the invention is a substance which is capable of forming a cohesive, solid or gelatinous film or layer. The film or layer in particular can be formed by casting or otherwise applying a formulation containing the film-forming substance solved or dispersed in a solvent and optionally further ingredients onto a surface. Preferably, the film-forming substance is a polymer.

A "disintegration agent" according to the invention is an agent which promotes or improves the dissolution or disintegration of the orodispersible film in the oral cavity. Disintegration agents in particular are hygroscopic agents and/or water-swellable agents. Preferably, disintegration agents are polymers.

A "softener" according to the invention is an agent which increases the flexibility, plasticity or fluidity of the orodispersible film.

The percentage amounts of ingredients defined herein in general refer to weight-per-weight percentages. All values given herein are approximate values and may differ from the actual stated value by up to 5 %, preferably by up to 2 % or up to 1 %.

In a first aspect, the present invention provides an orodispersible film comprising a base layer and a top layer attached onto the base layer, wherein the base layer comprises at least one first film-forming substance, and wherein the top layer comprises at least one second film-forming substance and at least one oral applicable, therapeutically active agent.

In a preferred embodiment, the orodispersible film is obtainable by casting and drying a base formulation to form the base layer, wherein the base formulation comprises the at least one first film-forming substance and a first solvent and does not comprise a therapeutically active agent; and applying a top formulation onto the base layer to form the top layer, wherein the top formulation comprises the at least one second film-forming substance, the at least one oral applicable, therapeutically active agent and a second solvent. Usually, the second solvent is either removed by drying to values acceptable for oral intake and/or is soaked into the base layer without further drying.

In preferred embodiments, the at least one first film-forming substance has a lower solubility in the second solvent than the at least one second film-forming substance. In particular, the solubility of the at least one first film-forming substance in the second solvent is lower, preferably at least 2 times lower, at least 5 times lower, at least 10 times lower, at least 25 times lower, or at least 100 times lower than the solubility of the at least one second film-forming substance. According to the present invention, the solubility preferably refers to the maximum amount of the substance which is soluble in the solvent in weight (substance) per volume (solvent) at a given temperature, preferably at 20°C or 25°C. In certain embodiments, the solubility of the substance is determined 10 minutes, preferably 5 minutes, 3 minutes, 2 minutes or 1 minute after bringing the substance into contact with the solvent, optionally under agitation such as mixing. When the solubility of a substance in a given solvent is compared to the solubility of a second substance in said solvent, preferably the solubility is determined under identical or highly similar conditions for both substances.

In preferred embodiments, the second solvent is an organic solvent or a mixture comprising one or more organic solvents. The mixture preferably comprises less than 50%, preferably less than 20%, more preferably less than 10% water. The organic solvent preferably is a polar organic solvent and in particular is miscible with water. The organic solvent is preferably selected from the group comprising highly volatile organic solvents, such as ethyl acetate, acetone, alcohols, in particular short-chain alcohols such as ethanol. Short-chain alcohols are alcohols having 1 to 6 carbon atoms, in particular 2, 3 or 4 carbon atoms, such as methanol, ethanol, and propanol including 1-propanol and 2-propanol, preferably ethanol. The organic solvent may also be a mixture of different organic solvents.

The first solvent preferably is water or a mixture comprising water. Preferably, the first solvent comprises at least 10 %, more preferably at least 20 %, at least 30 %, at least 50 % or at least 75 % water.

In further preferred embodiments, the at least one second film-forming substance provides a strong adhesion of the top layer to the base layer. A strong adhesion in this respect particularly refers to a high adhesive force or high fracture resistance between the top layer and the base layer. The adhesive force or fracture resistance can be determined by peel test which for example consists in measuring the force needed for tearing two adherent layers one from another. Said force is directly correlated to the adhesive force or fracture resistance between the top layer and the base layer. In particular, the adhesive force between the top layer and the base layer preferably is that strong, that the two layers cannot be separated without being damaged.

In preferred embodiments, the at least one first film-forming substance is a polymer. The first film-forming substance preferably is pharmaceutically acceptable. It is in particular selected from the group consisting of cellulose and derivatives, such as hydroxypropylmethylcellulose, polyvinylalcohols, starch, starch derivatives, modified starch such as maltodextrin, polyethylene glycol, polyvinyl pyrrolidone and its copolymers, alginates, and glycomannans. In certain preferred embodiments, the first film-forming substance is hydroxypropylmethylcellulose having an amount of hydroxypropyl of 5 to 15 %, an amount of methyl of 25 to 35 %, a viscosity of about 6 mPa*s as a 2% aqueous solution at 20°C. The amounts of hydroxypropyl and methyl refer to the relative amounts in relation to the monomeric units of the polymer. Thus, in a polymer comprising 10 % hydroxypropyl, 10 % or 1 out of 10 of the monomeric units of the polymer comprise a hydroxypropyl substituent. Particular examples of suitable first film-forming substances are Pharmacoat 606, Methocel E3 and Methocel E5. Preferably, the at least one first film-forming substance is present in the base layer in an amount of 50 to 75 %, preferably 55 to 65 %, more preferably 60 to 63 % of the weight of the solids content of the base layer. The at least one first film-forming substance preferably has a high solubility in aqueous solvents, in particular in solvents comprising at least 10 %, preferably at least 25 %, more preferably at least 50 % water.

In preferred embodiments, the at least one second film-forming substance is a polymer. The second film-forming substance preferably is pharmaceutically acceptable. It is preferably selected from the group consisting of hydroxypropylcellulose, hydroxyethylcellulose, crosslinked polyvinyl pyrollidone, ethyl cellulose, and poly methacrylates. In certain preferred embodiments, the second film-forming substance is hydroxypropylcellulose having a viscosity of about 5 to 7 mPa*s as a 2% aqueous solution at 25°C, and/or a weight-average molecular weight of about 70 to 90 kDa. In further preferred embodiments, the second film-forming substance is hydroxypropylcellulose having a viscosity of about 10 to 12 mPa*s as a 2% aqueous solution at 25°C, and/or a weight-average molecular weight of about 120 to 160 kDa. Particular examples of suitable second film-forming substances are Klucel EX, Klucel EXF, Klucel JX and Klucel JXF. The second film-forming substance preferably is present in the top layer in an amount of 10 to 80 %, preferably 25 to 70 %, more preferably 30 to 55 % of the weight of the solids content of the top layer. Preferably, the second film-forming substance has a high solubility in organic solvents or mixture comprising organic solvents, wherein the mixture comprises less than 50%, preferably less than 20%, more preferably less than 10% water. The organic solvent preferably is an organic solvent as defined above, in particular a short-chain alcohol such as ethanol.

In certain preferred embodiments, the at least one first film-forming substance is hydroxypropylmethylcellulose, in particular hydroxypropylmethylcellulose as defined above; and the at least one second film-forming substance is hydroxypropylcellulose, in particular hydroxypropylcellulose as defined above. Furthermore, in preferred embodiments the first solvent is water or a mixture comprising at least 20 % water, and the second solvent is a short-chain alcohol or a mixture comprising a short-chain alcohol and comprising less than 10 % water, preferably a short-chain alcohol as defined above.

The base layer of the orodispersible film may further comprise a disintegration agent. The disintegration agent preferably is a polymer and in particular is selected from the group consisting of hygroscopic agents such as polyvinyl pyrrolidone and polyethylene glycol, croscarmellose sodium, sodium alginate and rice starch, sodium starch glycolate, Aerosil, hydrogen carbonates in combination with acids. A particular example of the disintegration agent is polyvinyl pyrrolidone having an average particle size of less than 20 µm and/or an average nitrogen BET surface of more than 5 m²/g. Particular examples of suitable disintegration agents are Kollidon CL-M and Kollidon CL-SF. The disintegration agent preferably is present in the base layer in an amount of 5 to 40 %, preferably 10 to 30 %, more preferably 15 to 25 % of the solids content of the base layer.

Furthermore, the base layer of the orodispersible film may further comprise a softener. The softener preferably is hydrophilic and/or water soluble and in particular is selected from the group consisting of glycerol, polyethylene glycol, propylene glycol and triethyl citrate, sorbitol, xylitol, 1,3-butandiole, isopropylpalmitate, dibutylsebacate, paraffin oil. A preferred example of the softener is glycerol. The softener preferably is present in the base layer in an amount of 5 to 40 %, preferably 10 to 30 %, more preferably 15 to 25 % of the solids content of the base layer.

The base layer of the orodispersible film may comprise further ingredients such as hydrophilizing agents like Brij 35, flavoring agents, odorants, aromatic agents and dyes. Furthermore, the base layer may contain residual amounts of the first solvent. In particular, the amount of the first solvent in the base layer is less than 25 %, preferably less than 20 % or less than 15 %, more preferably less than 10 %. Preferably, all ingredients of the base layer are pharmaceutically acceptable. In certain embodiments, the base layer is free or substantially free of a pharmaceutically active agent, in particular during manufacturing. Substantially free in this respect refers to an amount of a pharmaceutically active agent relative to the weight of the base layer which is below the amount of the pharmaceutically active agent in the top layer relative to the weight of the top layer. In particular, the relative amount in the base layer is at least 10 times lower, preferably at least 100 times lower, more preferably at least 1000 times lower than the relative amount in the top layer.

In a particular embodiment, the base layer of the orodispersible film comprises or consists of 50 to 65 % hydroxypropylmethylcellulose, in particular hydroxypropylmethylcellulose as defined above, 15 to 25 % polyvinyl pyrrolidone, in particular polyvinyl pyrrolidone as defined above, 14 to 22 % glycerol, and less than 12 % water.

In preferred embodiments, the base layer has a high stability during the application of the top layer. High stability in this context inter alia refers to a high form stability, e.g. that the base layer does not furl or form ripples or waves; to a high integrity, e.g. that the base layer does not significantly disintegrate or dissolute; to a high tensile strength, e.g. an ultimate tensile strength of at least 3 N; and/or to a low maximal tensibility, e.g. a maximal tensibility of less than 6 %. Furthermore, the base layer preferably has a fast dissolubility in the oral cavity. In particular, a segment of the base layer having 1 to 6 cm² is dissolved in human salvia or water in less than 10 minutes, preferably less than 5 minutes, less than 3 minutes, less than 2 minutes, less than 90 seconds or less than 60 seconds.

The top layer of the orodispersible film may comprise further ingredients such as disintegration agent, in particular disintegration agents as defined above, softeners, in particular softeners as defined above, hydrophilizing agents like Brij 35, flavoring agents, odorants, aromatic agents, taste masking agents and dyes. Furthermore, the top layer may contain residual amounts of the second solvent. In particular, the amount of the second solvent in the top layer is less than 25 %, preferably less than 20 % or less than 15 %, more preferably less than 10 %. Preferably, all ingredients of the top layer are pharmaceutically acceptable.

In a particular embodiment, the top layer of the orodispersible film comprises or consists of 25 to 75 % of the pharmaceutically active agent, 20 to 75 % hydroxypropylcellulose, in particular hydroxypropylcellulose as defined above, and less than 12 % ethanol.

The top layer may continuously or discontinuously be applied onto the base layer. In preferred embodiments, the top layer is applied to the base layer after the base layer has been cut into suitable sections for administration.

The pharmaceutically active agent may be any orally applicable active agent. In particular, the pharmaceutically active agent is suitable for oral application. Examples of suitable pharmaceutically active agents are anti-allergic agents, anti-arrhythmic agents, antibiotics, anti-diabetic agents, anti-epileptic agents, anti-histaminic agents, antitussiva, cardiotonic agents, diuretics, hypotensive agents, narcotics, neuro-muscle blockers and sexual hormones such as vasopressors. Particular examples are rasagiline, rasagiline mesylate and tadalafil. Furthermore, the pharmaceutically active agent may be suitable for oral hygiene such as menthol. The pharmaceutically active agent may be a mixture of different agents. The amount of the pharmaceutically active agent in the orodispersible film preferably is in the range of 0.001 to 10 mg/cm², more preferably 0.01 to 2 mg/cm².

The orodispersible film preferably has a thickness of from 25 to 200 µm, more preferably from 65 to 100 µm, and/or a weight of from 5 to 500 g/m², more preferably from 25 to 100 g/m². Preferably, at least 95 % of the particles in the orodispersible film have a size of less than 500 µm, more preferably less than 200 µm. The orodispersible film may consist only of the base layer and the top layer or may comprise additional layers.

The invention further provides the orodispersible film as defined herein for use in medicine or oral hygiene. The use in medicine in particular includes the prophylaxis and treatment of a disease in a patient which can suitably be treated with the pharmaceutically active agent. In case the pharmaceutically active agent is rasagiline or rasagiline mesylate, the disease to be treated is Parkinson's disease, in particular early Parkinson's disease. In case the pharmaceutically active agent is tadalafil, the disease to be treated is pulmonary arterial hypertension or erectile dysfunction.

In a second aspect, the present invention provides a base layer for use in an orodispersible film according to the first aspect of the invention, comprising at least one first film-forming substance.

The base layer preferably is a base layer as defined above, having the features as defined above for the base layer of the orodispersible film according to the present invention.

In a third aspect, the present invention provides the use of a base layer according to the second aspect of the invention for the preparation of an orodispersible film, wherein a top formulation comprising at least one second film-forming substance and at least one oral applicable, therapeutically active agent is applied to the base layer.

In a fourth aspect, the present invention provides a method for preparing an orodispersible film, comprising the step of applying a top formulation comprising at least one second film-forming substance and at least one oral applicable, therapeutically active agent to the base layer according to the second aspect of the invention.

After application, the top formulation forms a top layer. The application of the top formulation may be by printing, in particular using flexoprint methods, or by any other means such as spraying or coating.

The base layer, the orodispersible film and the top layer preferably are as defined above, having the features as defined above.

### FIGURES

**Figure 1****:** Schematic overview of printing (flexography) technology
**Figure 2****:** ODFs printed with Tadalafil: unprinted ODF (a); ODF after one (b), two (c), three (d) and four (e) printings of a top layer
**Figure 3****:** Scanning electron micrograph of ODFs printed with Rasagiline mesylate (a) and Tadalafil (b)
**Figure 4****:** Polarised micrograph of ODFs printed with Rasagiline mesylate (a) and Tadalafil (b)
**Figure 5****:** X-ray diffractograms: a) 1 - Rasagiline mesylate; 2 - ODF printed with Rasagiline mesylate; b) 1 - Tadalafil; 2 - ODF printed with Tadalafil

### EXAMPLES

### 1. Materials and Methods

### 1.1 Manufacturing of the basic drug-free ODF

The ingredients of the basic drug-free ODF were Pharmacoat^{®} 606 or Methocel^{®} E5, Kollidon® CLM, Glycerol and water. ODFs were casted onto an intermediate liner using a coating machine equipped with a comma blade and conveyed through an oven with four heating-zones. The film was rolled up to a jumbo roll and cut into daughter rolls with a width of 2 cm and a length up to 100 m.

### 1.2 API printing on ODFs

The ingredients of the ink were Klucel® EX, brilliant blue and ethanol. Rasagiline mesylate and Tadalafil were used as model drugs, whereby Rasagiline mesylate is soluble in the ink, whereas Tadalafil forms an ink-suspension.

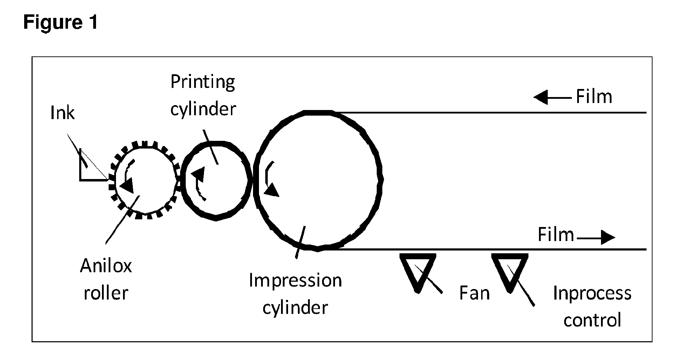

Flexography was used as printing method (Figure 1). The machine was equipped with an anilox roller with a capacity of 11.71 cm³/m² or 80 cm³/m². Each ODF was printed for four times with a printing speed of 16 m/min. Final film size was 2 cm to 3 cm.

### 1.3 API content

API content was determined using validated HPLC in-house methods after each printing (n = 6, n = 10 after the fourth printing).

### 1.4 Disintegration time

Disintegration time was recorded after each printing using three methods: the Petri dish method, the Slide frame method and a recently introduced method using a special sample holder for the pharmacopoeial disintegration tester (n = 6 for each method). [3, 4]

### 1.5 Mechanical properties

Mechanical properties were tested after each printing (n = 6) according to DIN EN ISO. [5, 6]

### 1.6 Film thickness

Film thickness was determined after each printing (n = 6) using a micrometer screw.

### 1.7 X-ray diffraction

X-ray diffractograms were collected for the drug-free film, the APIs and the ODFs after the fourth printing process using a X'Pert MDP PW3040/00 DY 653 (PANalytical B.B., Almelo, Netherland).

### 1.8 Visualisation of ODF properties

Polarised micrographs and scanning electron micrographs were collected for the drug-free film and the ODFs after the fourth printing process using a DMLB microscope (Leica Microsystems, Cambridge, UK) and a LEO 1430 VP (Leo Elektron Microscopy, Cambridge, UK).

Near Infrared Chemical Images were recorded for the drug-free film and after each printing process using the NIR-Cl 2450 (Malvern Instruments, Workestershire, UK).

### 2. Results and Discussion

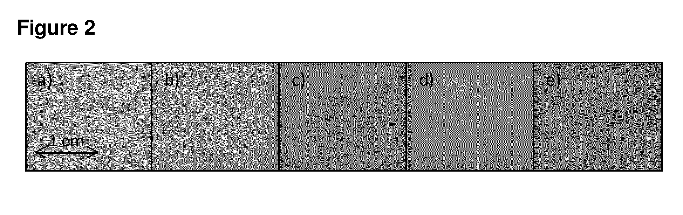

The introduced Flexography technology enabled the drug printing on ODFs. The films did not disintegrate or crumble during the process. Figure 2 shows ODFs printed with a coloured Tadalafil ink. The blue colour of brilliant blue is distributed homogeneously over the ODF.

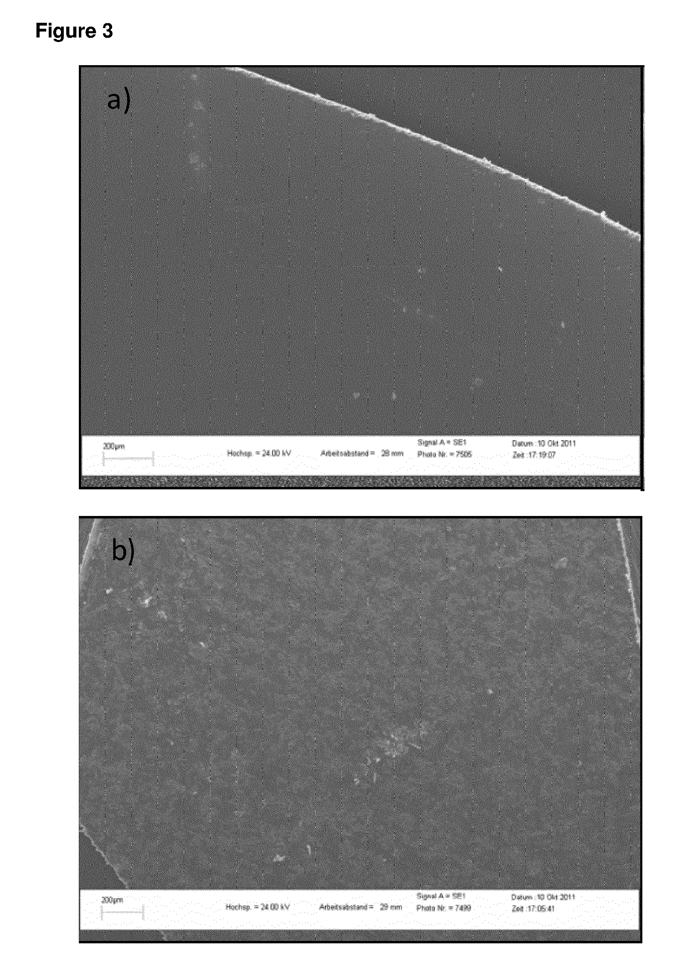

The film surface was still smooth and even. Homogeneous distribution of the API was proven by NIR-Cl. Figure 3 shows scanning electron micrographs of printed ODFs. The surface of ODFs printed with Tadalafil is not as even as the surface of ODFs printed with Rasagiline mesylate, because Tadalafil was printed in suspended form.

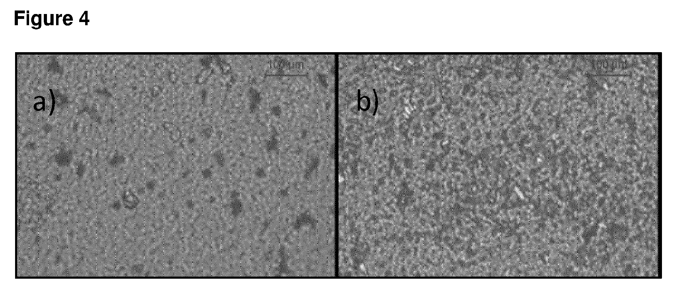

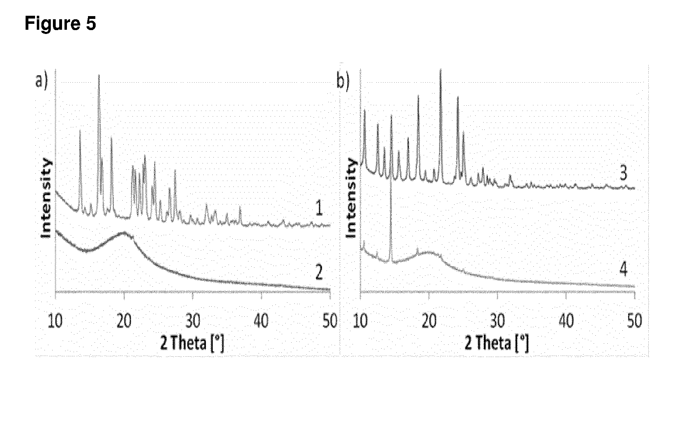

Rasagiline mesylate did not recrystallise after the printing process. No crystals were found by polarised microscopy (Figure 4) or X-ray diffraction (Figure 5), while Tadalafil crystals were detectable. The adherence of the crystals to the ODF is assumed to be strong enough to avoid loss of API during packaging and storage.

Film thicknesses increased with the number of applied printing processes. Increase was not significant for the Rasagiline mesylate printed ODFs. Further, it was not relevant for further processing and practical use.

Maximum force and maximum strain before rupture of the ODFs was not affected by printing. Maximum force was between 5 and 7 N. Maximum strain ranged from 2 to 5.5 %. Tensile strength was between 15 and 20 MPa.

Disintegration time was not affected by printing. All ODFs disintegrated within 45 s, irrespective on the used method.

The amount of printed API was dependent of the capacity of the anilox roller (Table 1). Surprisingly, using an anilox roller with a lower capacity led to a higher amount of transferred API. Further, standard deviation was much better. Using the 11.71 cm³/m² anilox roller, the transferred amount was identical for the printing of the suspension and the solution.

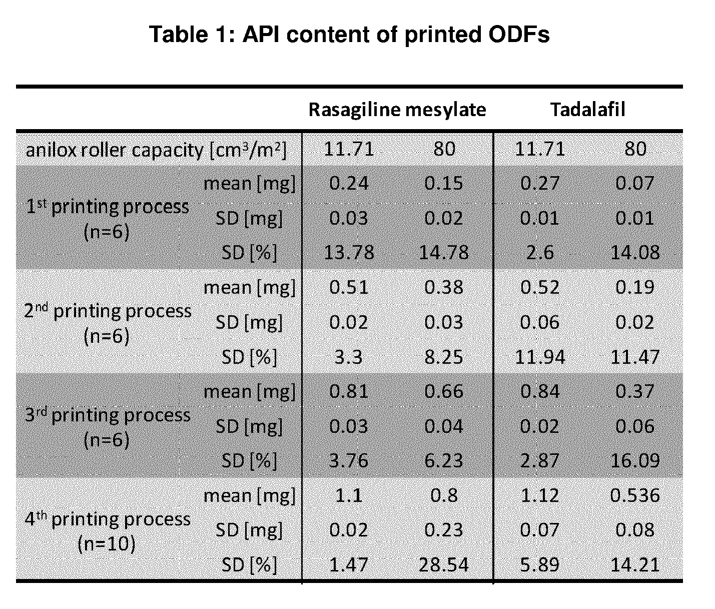

### 3. Conclusion

Printing on ODFs has been made possible and hence, a versatile new process for the manufacturing of ODFs with highly potent low-dose or heat sensitive drugs is introduced by our investigations. Flexography enables the printing of API solutions as well as suspensions obtaining a homogenous distribution of the API on the film. Flexography is feasible for small scale printing i.e. for individualised medicine as well as for industrial large scale processes.

### REFERENCES

[3] Hoffmann, EM et al., Development of new disintegration tests for orodispersible films, 3rd EuPFI conference, Strasbourg, France (2011)
[4] Hoffmann, EM et al., Comparison of different disintegration tests for oral soluble films, AAPS Annual Meeting, Washington DC, USA (2011)
[5] DIN EN ISO 527-1, Beuth Verlag GmbH, Berlin (1996)
[6] DIN EN ISO 527-3, Beuth Verlag GmbH, Berlin (2003)

## Claims

1. An orodispersible film (ODF) comprising a base layer and a top layer attached onto the base layer, wherein the base layer comprises at least one first film-forming substance, and wherein the top layer comprises at least one second film-forming substance and at least one oral applicable, therapeutically active agent.

2. The orodispersible film according to claim 1, obtainable by casting a base formulation to form the base layer, wherein the base formulation comprises the at least one first film-forming substance and a first solvent and does not comprise a therapeutically active agent; and applying a top formulation onto the base layer to form the top layer, wherein the top formulation comprises the at least one second film-forming substance, the at least one oral applicable, therapeutically active agent and a second solvent.

3. The orodispersible film according to claim 2, wherein the at least one first film-forming substance has a lower solubility in the second solvent than the at least one second film-forming substance.

4. The orodispersible film according to claim 2 or 3, wherein
(a) the solubility of the at least one first film-forming substance in the second solvent is at least 2 times lower, preferably at least 5 times lower, at least 10 times lower, at least 25 times lower, or at least 100 times lower than the solubility of the at least one second film-forming substance; and/or
(b) the second solvent is a volatile organic solvent or a mixture comprising an organic solvent, wherein the mixture comprises less than 50%, preferably less than 20%, more preferably less than 10% water, and wherein the organic solvent is preferably selected from the group comprising ethyl acetate, acetone, alcohols, in particular short-chain alcohols such as ethanol;
(c) the second solvent is an organic solvent or a mixture comprising an organic solvent, wherein the mixture comprises less than 50%, preferably less than 20%, more preferably less than 10% water, and wherein the organic solvent is preferably selected from the group comprising pharmaceutically acceptable non-volatile liquids, such as natural and synthetic oils.

5. The orodispersible film according to any one of claims 1 to 4, wherein the at least one second film-forming substance provides a strong adhesion of the top layer to the base layer and in particular is compatible with the at least one first film-forming substance.

6. The orodispersible film according to any one of claims 1 to 5, wherein the at least one first film-forming substance has one or more of the following characteristics:
(a) it is selected from the group consisting of celluloses and derivatives, such as hydroxypropylmethylcellulose, polyvinylalcohols, starch, starch derivatives, modified starch such as maltodextrin, polyethylene glycol, polyvinyl pyrrolidone and copolymers, alginates, and glycomannans;
(b) it is present in the base layer in an amount of 50 to 75 %, preferably 55 to 65
%, more preferably 60 to 63 % of the weight of the solids content of the base layer;
(c) it has a high solubility in aqueous solvents, in particular in solvents comprising at least 10 %, preferably at least 25 %, more preferably at least 50 % water;
(d) it is hydroxypropylmethylcellulose having an amount of hydroxypropyl of 5 to 15 %, an amount of methyl of 25 to 35 %, a viscosity of about 6 mPa*s as a 2% aqueous solution at 20°C.

7. The orodispersible film according to any one of claims 1 to 6, wherein the at least one second film-forming substance has one or more of the following characteristics:
(a) it is selected from the group consisting of hydroxypropylcellulose, hydroxyethylcellulose, ethylcellulose, crosslinked polyvinyl pyrrolidone, and polymethacrylates;
(b) it is present in the top layer in an amount of 10 to 80 %, preferably 25 to 70 %, more preferably 30 to 55 % of the weight of the solids content of the top layer;
(c) it has a high solubility in organic solvents or mixture comprising organic solvents, wherein the mixture comprises less than 50%, preferably less than 20%, more preferably less than 10% water, and wherein the organic solvent is preferably selected from the group comprising ethyl acetate, acetone, alcohols, in particular short-chain alcohols such as ethanol, and non-volatile liquids, such as natural or synthetic oils;
(d) it is hydroxypropylcellulose having a viscosity of about 5 to 7 mPa*s as a 2% aqueous solution at 25°C, and/or a weight-average molecular weight of about 70 to 90 kDa, or having a viscosity of about 10 to 12 mPa*s as a 2% aqueous solution at 25°C, and/or a weight-average molecular weight of about 120 to 160 kDa.

8. The orodispersible film according to any one of claims 1 to 7, wherein
(a) the at least one first film-forming substance is hydroxypropylmethylcellulose;
and
(b) the at least one second film-forming substance is hydroxypropylcellulose.

9. The orodispersible film according to any one of claims 2 to 8, wherein
(a) the first solvent is water or a mixture comprising at least 20 % water, and
(b) the second solvent is a short-chain alcohol or a mixture comprising a short-chain alcohol and comprising less than 10 % water.

10. The orodispersible film according to any one of claims 1 to 9, wherein the base layer further comprises a disintegration agent and/or a softener, wherein the disintegration agent preferably is selected from the group consisting of hygroscopic agents such as polyvinyl pyrrolidone and polyethylene glycol, croscarmellose sodium, sodium alginate, rice starch, sodium starch glycolate, Aerosil and hydrogen carbonates in combination with acids, and wherein the softener preferably is selected from the group consisting of glycerol, polyethylene glycol, propylene glycol, triethyl citrate, sorbitol, xylitol, 1,3-butandiole, isopropylpalmitate, dibutylsebacate and paraffin oil.

11. The orodispersible film according to any one of claims 1 to 10 for use in medicine or oral hygiene.

12. A base layer for use in an orodispersible film according to any one of claims 1 to 11, comprising at least one first film-forming substance.

13. The base layer according to claim 12, further comprising any one or more of the features defined in any one of claims 2 to 10.

14. Use of a base layer according to claim 12 or 13 for the preparation of an orodispersible film, wherein a top formulation comprising at least one second film-forming substance and at least one oral applicable, therapeutically active agent is applied to the base layer.

15. Method for preparing an orodispersible film, comprising the step of applying a top formulation comprising at least one second film-forming substance and at least one oral applicable, therapeutically active agent to the base layer according to claim 12 or 13.

16. The use according to claim 14 or the method according to claim 15, wherein the orodispersible film according to any one of claims 1 to 11 is prepared.
